# NOUVEAU FASCICULE DE BREVET EUROPEEN

(11) **EP 2 135 861 B2**
(45) Date de publication et mention de la décision concernant l'opposition: **16.12.2020**
(45) Mention de la délivrance du brevet: 30.07.2014
(21) Numéro de dépôt: 09290466.3
(22) Date de dépôt: 19.06.2009
(51) Int. Cl.: C07D 223/16, C07B 37/10, C07B 43/06

(54) **Procédé de synthèse de la 7,8-diméthoxy-1,3-dihydro-2H-3-benzazépin-2-one, et son application à la synthèse de l'ivabradine et de ses sels d'addition avec un acide pharmaceutiquement acceptable**
Verfahren zur Herstellung von 7,8-Dimethoxy-1,3-Dihydro-2H-3-Benzazepin-2-on und seine Anwendung bei der Synthese von Ivabradin sowie dessen Additionssalze einer pharmazeutisch unbedenklichen Säure
Process for the synthesis of the 7,8-dimethoxy-1,3-dihydro-2H-3-benzazepin-2-one, and its application to the synthesis of ivabradine as well as its addition salts with a pharmaceutically acceptable acid

(30) Priorité: 20.06.2008 FR 0803452
(43) Date de publication de la demande: 23.12.2009
(73) Titulaire: Les Laboratoires Servier, 92284 Suresnes Cedex (FR)
(72) Inventeur: Lerestif, Jean-Michel, 76190 Yvetot (FR); Lecouve, Jean-Pierre, 76600 Le Havre (FR); Brigot, Daniel, 76190 Ste Marie des Champs (FR)

(56) Documents cités:
- EP-A- 0 161 604
- EP-A- 0 534 859
- WO-A-2005/110993
- WO-A-2007/011820
- BOMHARD A ET AL: "SPECIFIC BRADYCARDIC AGENTS. 2. HETEROAROMATIC MODIFICATIONS IN THE SIDE CHAIN OF SPECIFIC BRADYCARDIC BENZAZEPINONES: CHEMISTRY, PHARMACOLOGY, AND STRUCTURE-ACTIVITY RELATIONSHIPS" JOURNAL OF MEDICINAL CHEMISTRY, AMERICAN CHEMICAL SOCIETY. WASHINGTON.; US, vol. 34, 1 janvier 1991 (1991-01-01), pages 942-947, XP002277379 ISSN: 0022-2623
- REIFFEN M ET AL: "SPECIFIC BRADYCARDIC AGENTS. 1. CHEMISTRY, PHARMACOLOGY AND STRUCTURE-ACTIVITY RELATIONSHIPS OF SUBSTITUTED BENZAZEPINONES, A NEW CLASS OF COMPOUNDS EXERTING ANTIISCHEMIC PROPERTIES" JOURNAL OF MEDICINAL CHEMISTRY, AMERICAN CHEMICAL SOCIETY. WASHINGTON.; US, vol. 33, no. 5, 1 mai 1990 (1990-05-01), pages 1496-1504, XP002047919 ISSN: 0022-2623

## Description

La présente invention concerne un procédé de synthèse de la 7,8-diméthoxy-1,3-dihydro-2*H*-3-benzazépin-2-one de formule (I), et son application à la synthèse de l'ivabradine, de ses sels d'addition à un acide pharmaceutiquement acceptable et des leurs hydrates.

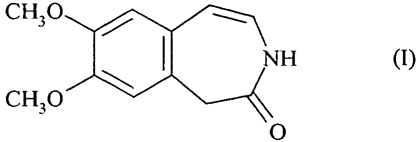

Le composé de formule (I) obtenu selon le procédé de l'invention est utile dans la synthèse de l'ivabradine de formule (II) 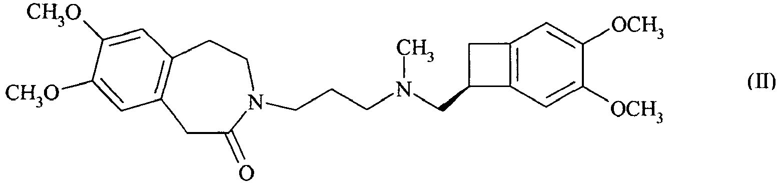 ou 3-{3-[{[(7*S*)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

Ce brevet décrit la synthèse du chlorhydrate de l'ivabradine à partir du composé de formule (III) : 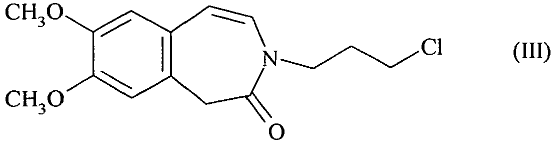 et fait référence à la publication J. Med. Chem 1990, Vol. 33 (5), 1496-1504 pour la préparation de ce composé.

La voie de synthèse du composé de formule (III) décrite dans cette publication utilise une réaction d'alkylation du composé de formule (I) :

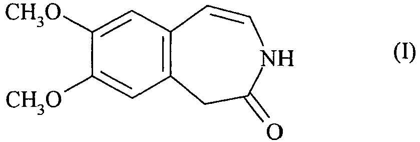

La publication précitée décrit la préparation du composé de formule (I) en utilisant comme intermédiaire le *N*-(2,2-diméthoxyéthyl)-2-(3,4-diméthoxyphényl)-acétamide obtenu à partir de l'acide (3,4-diméthoxyphényl) acétique. La cyclisation du phénylacétamide obtenu se fait en présence de l'acide chlorhydrique dans l'acide acétique, pour conduire au composé de formule (I) avec un rendement global de 58% par rapport à l'acide (3,4-diméthoxyphényl)acétique.

La publication J. Med. Chem. 1991, vol. 34(3), 942-947 et la demande de brevet européen EP 0 161 604 décrivent la synthèse du composé de formule (I) dans laquelle les composés intermédiaires sont isolés.

Compte-tenu de l'intérêt industriel de l'ivabradine et de ses sels, il était impératif de trouver un procédé performant permettant notamment d'accéder à la 7,8-diméthoxy-1,3-dihydro-2*H*-3-benzazépin-2-one de formule (I) avec un excellent rendement.

Or, la Demanderesse s'est aperçue que de manière surprenante, en utilisant des conditions opératoires spécifiques, il était possible d'obtenir à l'échelle industrielle le composé de formule (I) avec un rendement supérieur à 92% et une pureté chimique supérieure à 99,5%.

Plus spécifiquement, la présente invention concerne un procédé de synthèse du composé de formule (I) : 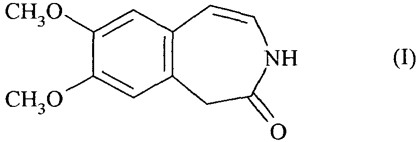 caractérisé en ce que l'acide (3,4-diméthoxyphényl) acétique de formule (IV) : 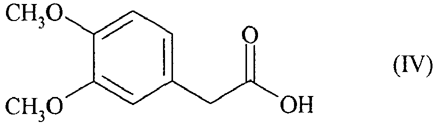 est transformé en composé de formule (V) : 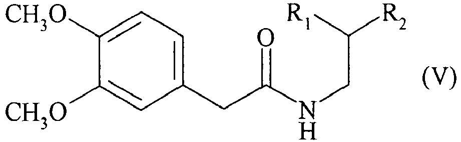 dans laquelle les groupements R₁ et R₂, identiques ou différents, représentent des groupements alkoxy (C₁-C₆) linéaires ou ramifiés, ou bien forment ensemble avec l'atome de carbone qui les porte un cycle 1,3-dioxane, 1,3-dioxolane ou 1,3-dioxépane, lequel n'est pas isolé et est soumis à une réaction de cyclisation en milieu acide pour conduire après isolement au composé de formule (I).

La transformation du composé de formule (IV) en composé de formule (V) est effectuée par transformation préliminaire du composé de formule (IV) en composé de formule (VI) : 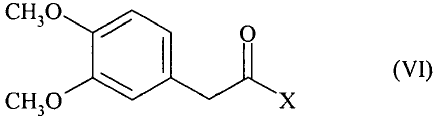 dans laquelle X représente un atome d'halogène ou un groupement OCOR₃ où R₃ est un groupement alkyle (C₁-C₆) linéaire ou ramifié, un groupement phényle, un groupement benzyle ou un groupement imidazole,
dans un solvant organique,
lequel composé de formule (VI) n'est pas isolé et est soumis à une réaction de condensation avec un composé de formule (VII) : 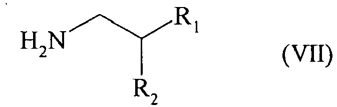 dans laquelle les groupements R₁ et R₂, identiques ou différents, représentent des groupements alkoxy (C₁-C₆) linéaires ou ramifiés, ou bien forment ensemble avec l'atome de carbone qui les porte un cycle 1,3-dioxane, 1,3-dioxolane ou 1,3-dioxépane,
en présence d'une base dans un solvant organique,
pour conduire au composé de formule (V) :

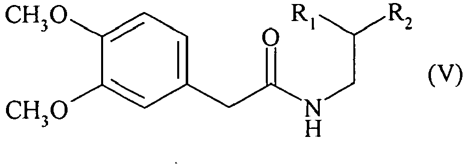

Le groupement X dans le composé de formule (VI) représente préférentiellement un atome de chlore.

Parmi les solvants organiques pouvant être utilisés pour la réaction de transformation du composé de formule (IV) en composé de formule (VI), on peut citer à titre non limitatif le toluène, le dichlorométhane, le 2-méthyltétrahydrofurane, le chlorobenzène, le 1,2-dichloroéthane, le chloroforme et le dioxane.

Le solvant organique préféré pour la réaction de transformation du composé de formule (IV) en composé de formule (VI) est le dichlorométhane.

La température de la réaction de transformation du composé de formule (IV) en composé de formule (VI) est préférentiellement comprise entre 20 et 40°C.

Le réactif préférentiellement utilisé pour réaliser la transformation du composé de formule (IV) en composé de formule (VI) pour lequel X représente un atome de chlore est le chlorure de thionyle.

La quantité de chlorure de thionyle engagée dans la réaction de transformation du composé de formule (IV) en composé de formule (VI) est préférentiellement comprise entre 1 et 1,3 moles par mole de composé de formule (IV).

Parmi les solvants organiques pouvant être utilisés pour la réaction entre le composé de formule (VI) et le composé de formule (VII), on peut citer à titre non limitatif le toluène, le dichlorométhane, le 2-méthyltétrahydrofurane, le chlorobenzène, le 1,2-dichloroéthane, le chloroforme et le dioxane.

Le solvant organique préféré pour la réaction entre le composé de formule (VI) et le composé de formule (VII) est le dichlorométhane.

La température de la réaction entre le composé de formule (VI) et le composé de formule (VII) est préférentiellement comprise entre 0 et 40°C.

La quantité de composé de formule (VII) engagée dans la réaction avec le composé de formule (VI) est préférentiellement comprise entre 1 et 1,2 moles par mole de composé de formule (VI).

La quantité de base engagée dans la réaction entre le composé de formule (VI) et le composé de formule (VII) est préférentiellement comprise entre 1 et 1,3 moles par mole de composé de formule (VI).

Parmi les bases qui peuvent être utilisées pour la réaction entre le composé de formule (VI) et le composé de formule (VII), on peut citer à titre non limitatif la pyridine, la DMAP et les amines tertiaires, par exemple la triéthylamine, la DIEA, la N-méthylpipéridine, la DBU, le DABCO, le DBN et la N-méthylmorpholine.

La base préférentiellement utilisée pour la réaction entre le composé de formule (VI) et le composé de formule (VII) est la triéthylamine.

Parmi les acides pouvant être utilisés pour effectuer la cyclisation du composé de formule (V) en composé de formule (I), on peut citer à titre non limitatif l'acide sulfurique concentré, l'acide polyphosphorique, l'acide chlorhydrique concentré en solution aqueuse, l'acide chlorhydrique concentré en solution dans l'acide acétique, l'acide bromhydrique concentré en solution dans l'acide acétique et l'acide méthanesulfonique.

La quantité d'acide engagée dans la réaction de cyclisation du composé de formule (V) en composé de formule (I) est préférentiellement comprise entre 5 et 15 moles par mole de composé de formule (V).

La température de la réaction de cyclisation en milieu acide du composé de formule (V) est préférentiellement comprise entre 0 et 40°C.

L'acide préférentiellement utilisé pour effectuer la cyclisation du composé de formule (V) en composé de formule (I) est l'acide sulfurique concentré.

La quantité d'acide sulfurique concentrée engagée dans la réaction de cyclisation du composé de formule (V) est préférentiellement comprise entre 1,5 et 3 millilitres par gramme d'acide (3,4-diméthoxyphényle) acétique de formule (IV).

Le composé de formule (I) obtenu selon le procédé de la présente invention est particulièrement utile comme intermédiaire de synthèse dans la synthèse de l'ivabradine, de ses sels d'addition à un acide pharmaceutiquement acceptable et de ses hydrates.

A titre d'exemple, l'alkylation du composé de formule (I) par un composé de formule (VIII) : 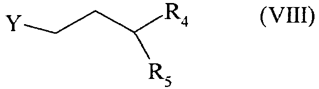 dans laquelle R₄ et R₅, identiques ou différents, représentent chacun un groupement alkoxy (C₁-C₆) linéaire ou ramifié, ou bien forment ensemble avec l'atome de carbone qui les porte un cycle 1,3-dioxane ou 1,3-dioxolane, et Y représente un atome d'halogène, préférentiellement un atome de brome, ou un groupement tosylate, mésylate ou triflate,
conduit au composé de formule (IX) 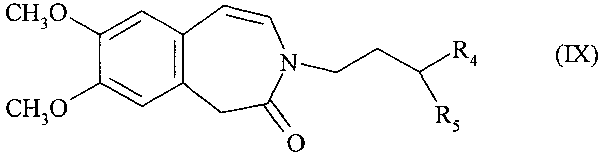 dont l'hydrogénation catalytique conduit au composé hydrogéné correspondant de formule (X) : 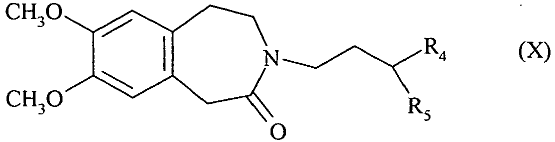 dans laquelle R₄ et R₅ sont tels que définis dans la formule (VIII),
dont la déprotection du diacétal conduit à l'aldéhyde de formule (XI) : 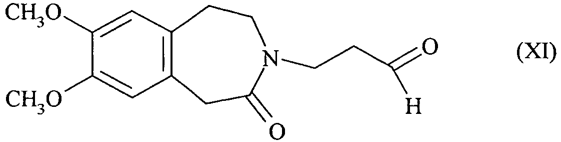 qui est mis en réaction avec la (7*S*)-3,4-diméthoxybicyclo[4.2.0]octa-1,3,5-trién-7-yl]-*N-*méthylméthanamine dans des conditions d'amination réductrice, pour conduire à l'ivabradine, qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

### Lexique des abréviations utilisées :

BOP : benzotriazol-1-yl-oxy-tris-(diméthylamino)-phosphonium hexafluorophosphate
CDI : carbonyldiimidazole
DABCO : 1,4-diazabicyclo[2.2.2]octane
DBN : 1,5-diazabicyclo[4.3.0]non-5-ène
DBU : 1,8-diazabicyclo[5.4.0]undec-7-ène
DCC : dicyclohexylcarbodiimide
DIEA : *N,N*-diisopropyléthylamine
DMAP : 4-diméthylaminopyridine
EDCI: 1-(3-diméthylaminopropyl)-3-éthyl-carbodiimide chlorhydrate
HATU: *O*-(7-azabenzotriazol-1-yl)-1,1,3,3-tétraméthyluronium hexafluorophosphate
HBTU: *O*-(benzotriazol-1-yl)-1,1,3,3-tétraméthyluronium hexafluorophosphate
HOAT: 1-hydroxy-7-azabenzotriazole
HOBT : 1-hydroxybenzotriazole
NHS : *N*-hydroxysuccinimide
NMP : *N*-méthylpyrrolidone
PyBOP : *O*-(benzotriazol-1-yl)-oxytripyrrolidinophosphonium hexafluorophosphate
TBTU : *O*-(benzotriazol-1-yl)-1,1,3,3-tétraméthyluronium tétrafluoroborate
T3P : *n*-propane phosphonic anhydride

L'exemple ci-dessous illustre l'invention.

### Préparation de la 7,8-diméthoxy-1,3-dihydro-2H-3-benzazépin-2-one

### Stade A : Chlorure de l'acide (3,4-diméthoxyphényl) acétique

Dans un réacteur, charger 135g d'acide (3,4-diméthoxyphényl) acétique et 270 ml de dichlorométhane puis amener la température du milieu réactionnel à reflux et ajouter goutte à goutte 90g de chlorure de thionyle. Agiter le mélange à reflux pendant 3 h. La solution obtenue est utilisée telle que pour l'étape suivante.

### Stade B : N-(2,2-diméthoxyéthyl)-2-(3,4-diméthoxyphényl)acétamide

Dans un réacteur, charger 225ml de dichlorométhane, 44,15g de 2,2-diméthoxyéthylamine et 44,35g de triéthylamine puis refroidir le milieu à 10°C et ajouter goutte à goutte 237,4g de la solution obtenue à l'étape précédente (correspondant à 75g d'acide (3,4-diméthoxyphényl) acétique) en maintenant la température masse à 10°C. Agiter le mélange 2h à 15°C. La solution obtenue est utilisée telle que pour l'étape suivante.

### Stade C : 7,8-diméthoxy-1,3-dihydro-2H-3-benzazépin-2-one

Dans un réacteur contenant la solution obtenue à l'étape précédente et refroidi à 10°C, ajouter 150ml d'acide sulfurique 36N en maintenant la température en dessous de 20°C. Agiter le mélange à 15-20°C pendant 10h puis laisser décanter le milieu réactionnel et recueillir la phase acide sulfurique contenant le produit.
Le produit est obtenu par précipitation dans un mélange eau/NMP (4/1), filtration et séchage avec un rendement de 92,9% par rapport à l'acide (3,4-diméthoxyphényl) acétique et une pureté chimique supérieure à 99,5%.

## Revendications

1. Procédé de synthèse du composé de formule (I) : 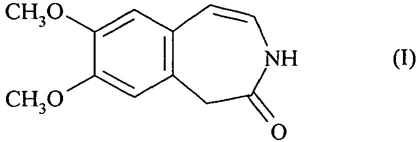 **caractérisé en ce que** l'acide (3,4-diméthoxyphényl) acétique de formule (IV) : 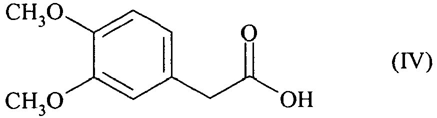 est transformé en composé de formule (V) : 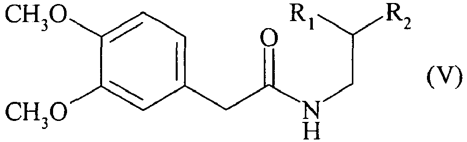 dans laquelle les groupements R₁ et R₂, identiques ou différents, représentent des groupements alkoxy (C₁-C₆) linéaires ou ramifiés, ou bien forment ensemble avec l'atome de carbone qui les porte un cycle 1,3-dioxane, 1,3-dioxolane ou 1,3-dioxépane,
par transformation préliminaire du composé de formule (IV) en composé de formule (VI) : 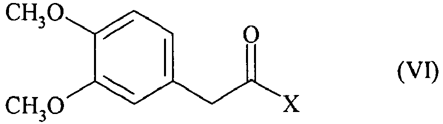 dans laquelle X représente un atome d'halogène ou un groupement OCOR₃ où R₃ est un groupement alkyle (C₁-C₆) linéaire ou ramifié, un groupement phényle, un groupement benzyle ou un groupement imidazolyle,
dans un solvant organique,
lequel composé de formule (VI) n'est pas isolé et est soumis à une réaction de condensation avec un composé de formule (VII) : 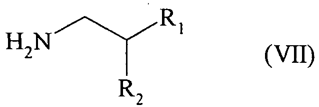 dans laquelle les groupements R₁ et R₂, identiques ou différents, représentent des groupements alkoxy (C₁-C₆) linéaires ou ramifiés, ou bien forment ensemble avec l'atome de carbone qui les porte un cycle 1,3-dioxane, 1,3-dioxolane ou 1,3-dioxépane,
en présence d'une base dans un solvant organique,
pour conduire au composé de formule (V) : 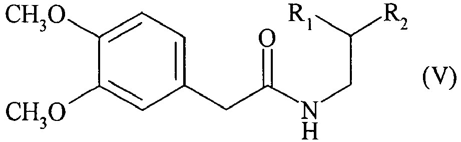 lequel n'est pas isolé et est soumis à une réaction de cyclisation en milieu acide pour conduire après isolement au composé de formule (I).

2. Procédé de synthèse selon la revendication 1, **caractérisé en ce que**, dans le composé de formule (VI), X représente un atome de chlore.

3. Procédé de synthèse selon l'une quelconque des revendications 1 à 2, **caractérisé en ce que** le solvant utilisé pour la transformation du composé de formule (IV) en composé de formule (VI) est le dichlorométhane.

4. Procédé de synthèse selon l'une quelconque des revendications 1 à 3, **caractérisé en ce que** la température de réaction de transformation du composé de formule (IV) en composé de formule (VI) est comprise entre 20°C et 40°C.

5. Procédé de synthèse selon l'une quelconque des revendications 1 à 4, **caractérisé en ce que** le réactif utilisé pour la transformation du composé de formule (IV) en composé de formule (VI) est le chlorure de thionyle.

6. Procédé de synthèse selon la revendication 5, **caractérisé en ce que** la quantité de chlorure de thionyle engagée dans la réaction de transformation du composé de formule (IV) en composé de formule (VI) est comprise entre 1 et 1.3 moles par mole de composé de formule (IV).

7. Procédé de synthèse selon l'une quelconque des revendications 1 à 6, **caractérisé en ce que** le solvant de la réaction entre les composés de formule (VI) et (VII) est le dichlorométhane.

8. Procédé de synthèse selon l'une quelconque des revendications 1 à 7, **caractérisé en ce que** la température de la réaction entre les composés de formule (VI) et (VII) est comprise entre 0 et 40°C.

9. Procédé de synthèse selon l'une quelconque des revendications 1 à 8, **caractérisé en ce que** la quantité de composé (VII) engagée dans la réaction avec le composé de formule (VI) est comprise entre 1 et 1.2 moles par mole de composé de formule (VI).

10. Procédé de synthèse selon l'une quelconque des revendications 1 à 9, **caractérisé en ce que** la quantité de base engagée dans la réaction entre les composés de formule (VI) et (VII) est comprise entre 1 et 1.3 moles par mole de composé (VI).

11. Procédé de synthèse selon l'une quelconque des revendications 1 à 10, **caractérisé en ce que** la base utilisée dans la réaction entre les composés de formule (VI) et (VII) est la pyridine, la DMAP ou une amine tertiaire.

12. Procédé de synthèse selon la revendication 11, **caractérisé en ce que** l'amine utilisée dans la réaction entre les composés de formule (VI) et (VII) est la triéthylamine.

13. Procédé de synthèse selon l'une quelconque des revendications 1 à 12, **caractérisé en ce que** la quantité d'acide engagée dans la réaction de cyclisation du composé de formule (V) est comprise entre 5 et 15 moles par mole de composé de formule (V).

14. Procédé de synthèse selon l'une quelconque des revendications 1 à 13, **caractérisé en ce que** la température de la réaction de cyclisation en milieu acide du composé de formule (V) est comprise entre 0 et 40°C.

15. Procédé de synthèse selon l'une quelconque des revendications 1 à 14, **caractérisé en ce que** l'acide utilisé pour la cyclisation du composé de formule (V) est l'acide sulfurique concentré.

16. Procédé de synthèse selon la revendication 15, **caractérisé en ce que** la quantité d'acide sulfurique concentré engagée dans la réaction de cyclisation du composé de formule (V) est comprise entre 1.5 et 3 millilitres par gramme d'acide (3,4-diméthoxyphényl) acétique de formule (IV).

17. Procédé de synthèse de l'ivabradine et de ses sels pharmaceutiquement acceptables, dans lequel le composé de formule (IV) est transformé en composé de formule (I) selon le procédé de la revendication 1, puis le composé de formule (I) est transformé en ivabradine.

## Patentansprüche

1. Verfahren zur Synthese der Verbindung der Formel (I): 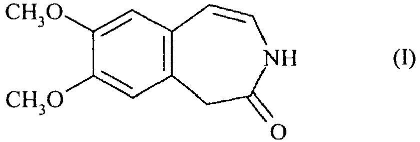 **dadurch gekennzeichnet, dass** man (3,4-Dimethoxyphenyl)-essigsäure der Formel (IV): 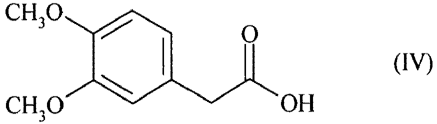 in die Verbindung der Formel (V): 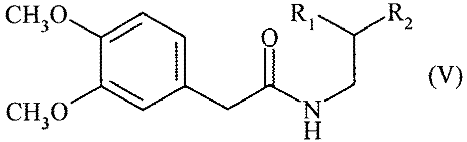 in der die Gruppen R₁ und R₂, die identisch oder verschieden sind, geradkettige oder verzweigte (C₁-C₆)-Alkoxygruppen bedeuten, oder gemeinsam mit dem sie tragenden Kohlenstoffatom einen 1,3-Dioxan-, 1,3-Dioxolan- oder 1,3-Dioxepan-Ring bilden, umwandelt,
durch vorausgehende Umwandlung der Verbindung der Formel (IV) in die Verbindung der Formel (VI): 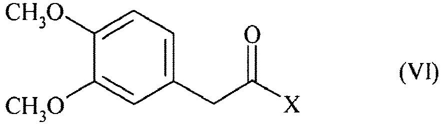 in der X ein Halogenatom oder eine Gruppe OCOR₃ bedeutet, worin R₃ eine geradkettige oder verzweigte (C₁-C₆)-Alkylgruppe, eine Phenylgruppe, eine Benzylgruppe oder eine Imidazolylgruppe bedeutet,
in einem organischen Lösungsmittel,
welche Verbindung der Formel (VI) nicht isoliert wird und einer Kondensationsreaktion mit einer Verbindung der Formel (VII): 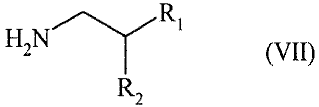 in der die Gruppen R₁ und R₂, die identisch oder verschieden sind, geradkettige oder verzweigte (C₁-C₆)-Alkoxygruppen bedeuten oder gemeinsam mit dem sie tragenden Kohlenstoffatom einen 1,3-Dioxan-, 1,3-Dioxolan- oder 1,3-Dioxepan-Ring bilden,
in Gegenwart einer Base in einem organischen Lösungsmittel unterworfen wird,
zur Bildung der Verbindung der Formel (V): 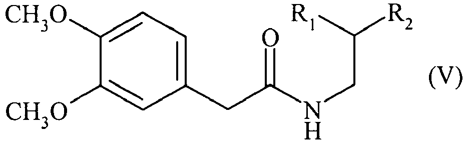 welche nicht isoliert wird und einer Cyclisierungsreaktion in saurem Medium unterworfen wird, sodass man nach der Isolierung die Verbindung der Formel (I) erhält.

2. Syntheseverfahren nach Anspruch 1, **dadurch gekennzeichnet, dass** in der Verbindung der Formel (VI) X ein Chloratom bedeutet.

3. Syntheseverfahren nach einem der Ansprüche 1 bis 2, **dadurch gekennzeichnet, dass** das zur Umwandlung der Verbindung der Formel (IV) in die Verbindung der Formel (VI) verwendete Lösungsmittel Dichlormethan ist.

4. Syntheseverfahren nach einem der Ansprüche 1 bis 3, **dadurch gekennzeichnet, dass** die Temperatur der Reaktion der Umwandlung der Verbindung der Formel (IV) in die Verbindung der Formel (VI) zwischen 20 °C und 40 °C beträgt.

5. Syntheseverfahren nach einem der Ansprüche 1 bis 4, **dadurch gekennzeichnet, dass** das für die Umwandlung der Verbindung der Formel (IV) in die Verbindung der Formel (VI) verwendete Reagens Thionylchlorid ist.

6. Syntheseverfahren nach Anspruch 5, **dadurch gekennzeichnet, dass** die bei der Reaktion der Umwandlung der Verbindung der Formel (IV) in die Verbindung der Formel (VI) verwendete Menge des Thionylchlorids zwischen 1 und 1,3 Mol pro Mol der Verbindung der Formel (IV) beträgt.

7. Syntheseverfahren nach einem der Ansprüche 1 bis 6, **dadurch gekennzeichnet, dass** das Lösungsmittel der Reaktion zwischen den Verbindungen der Formeln (VI) und (VII) Dichlormethan ist.

8. Syntheseverfahren nach einem der Ansprüche 1 bis 7, **dadurch gekennzeichnet, dass** die Temperatur der Reaktion zwischen den Verbindungen der Formeln (VI) und (VII) zwischen 0 und 40 °C beträgt.

9. Syntheseverfahren nach einem der Ansprüche 1 bis 8, **dadurch gekennzeichnet, dass** die Menge der bei der Reaktion der Verbindung der Formel (VI) verwendete Verbindung der Formel (VII) zwischen 1 und 1,2 Mol pro Mol der Verbindung der Formel (VI) beträgt.

10. Syntheseverfahren nach einem der Ansprüche 1 bis 9, **dadurch gekennzeichnet, dass** die Menge der bei der Reaktion der Verbindungen der Formeln (VI) und (VII) verwendeten Base zwischen 1 und 1,3 Mol pro Mol der Verbindung der Formel (VI) beträgt.

11. Syntheseverfahren nach einem der Ansprüche 1 bis 10, **dadurch gekennzeichnet, dass** die bei der Reaktion der Verbindungen der Formeln (VI) und (VII) verwendete Base Pyridin, DMAP oder ein tertiäres Amin ist.

12. Syntheseverfahren nach Anspruch 11, **dadurch gekennzeichnet, dass** das bei der Reaktion der Verbindungen der Formeln (VI) und (VII) verwendete Amin Triethylamin ist.

13. Syntheseverfahren nach einem der Ansprüche 1 bis 12, **dadurch gekennzeichnet, dass** die Menge der bei der Cyclisierungsreaktion der Verbindung der Formel (V) verwendete Säure zwischen 5 und 15 Mol pro Mol der Verbindung der Formel (V) beträgt.

14. Syntheseverfahren nach einem der Ansprüche 1 bis 13, **dadurch gekennzeichnet, dass** die Temperatur der Reaktion der Cyclisierung der Verbindung der Formel (V) in saurem Medium zwischen 0 und 40 °C beträgt.

15. Syntheseverfahren nach einem der Ansprüche 1 bis 14, **dadurch gekennzeichnet, dass** die für die Cyclisierung der Verbindung der Formel (V) verwendete Säure konzentrierte Schwefelsäure ist.

16. Syntheseverfahren nach Anspruch 15, **dadurch gekennzeichnet, dass** die Menge der bei der Reaktion der Cyclisierung der Verbindung der Formel (V) verwendeten Schwefelsäure zwischen 1,5 und 3 Milliliter pro Gramm (3,4-Dimethoxyphenyl)-essigsäure der Formel (IV) beträgt.

17. Verfahren zur Synthese von Ivabradin und seinen pharmazeutisch annehmbaren Salzen, worin die Verbindung der Formel (IV) nach dem Verfahren des Anspruchs 1 in die Verbindung der Formel (I) umgewandelt wird und dann die Verbindung der Formel (I) in Ivabradin überführt wird.

## Claims

1. Process for the synthesis of the compound of formula (I): 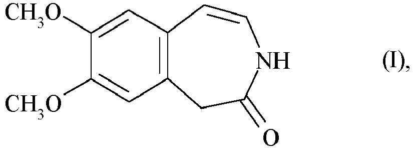 **characterised in that** (3,4-dimethoxyphenyl)acetic acid of formula (IV): 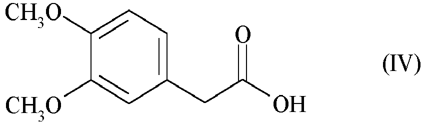 is converted into the compound of formula (V): 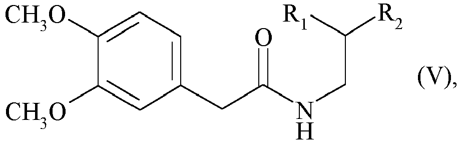 wherein the groups R₁ and R₂, which may be the same or different, represent linear or branched (C₁-C₆)alkoxy groups or, together with the carbon atom carrying them, form a 1,3-dioxane, 1,3-dioxolane or 1,3-dioxepane ring,
by means of preliminary conversion of the compound of formula (IV) into the compound of formula (VI): 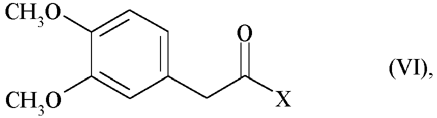 wherein X represents a halogen atom or a group OCOR₃ wherein R₃ is a linear or branched (C₁-C₆)alkyl group, a phenyl group, a benzyl group or an imidazolyl group,
in an organic solvent,
which compound of formula (VI) is not isolated and is subjected to a condensation reaction with a compound of formula (VII): 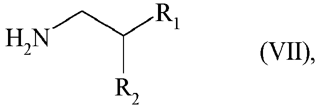 wherein the groups R₁ and R₂, which may be the same or different, represent linear or branched (C₁-C₆)alkoxy groups or, together with the carbon atom carrying them, form a 1,3-dioxane, 1,3-dioxolane or 1,3-dioxepane ring,
in the presence of a base in an organic solvent,
to yield the compound of formula (V): 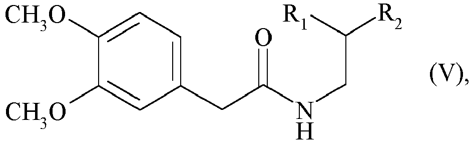 which is not isolated and is subjected to a cyclisation reaction in an acid medium to yield, after isolation, the compound of formula (I).

2. Synthesis process according to claim 1, **characterised in that**, in the compound of formula (VI), X represents a chlorine atom.

3. Synthesis process according to either claim 1 or claim 2, **characterised in that** the solvent used for conversion of the compound of formula (IV) into the compound of formula (VI) is dichloromethane.

4. Synthesis process according to any one of claims 1 to 3, **characterised in that** the temperature of the reaction for conversion of the compound of formula (IV) into the compound of formula (VI) is from 20°C to 40°C.

5. Synthesis process according to any one of claims 1 to 4, **characterised in that** the reagent used for conversion of the compound of formula (IV) into the compound of formula (VI) is thionyl chloride.

6. Synthesis process according to claim 5, **characterised in that** the amount of thionyl chloride used in the reaction for conversion of the compound of formula (IV) into the compound of formula (VI) is from 1 to 1.3 moles per mole of compound of formula (IV).

7. Synthesis process according to any one of claims 1 to 6, **characterised in that** the solvent for the reaction between the compounds of formulae (VI) and (VII) is dichloromethane.

8. Synthesis process according to any one of claims 1 to 7, **characterised in that** the temperature of the reaction between the compounds of formulae (VI) and (VII) is from 0 to 40°C.

9. Synthesis process according to any one of claims 1 to 8, **characterised in that** the amount of compound (VII) used in the reaction with the compound of formula (VI) is from 1 to 1.2 moles per mole of compound of formula (VI).

10. Synthesis process according to any one of claims 1 to 9, **characterised in that** the amount of base used in the reaction between the compounds of formulae (VI) and (VII) is from 1 to 1.3 moles per mole of compound (VI).

11. Synthesis process according to any one of claims 1 to 10, **characterised in that** the base used in the reaction between the compounds of formulae (VI) and (VII) is pyridine, DMAP or a tertiary amine.

12. Synthesis process according to claim 11, **characterised in that** the amine used in the reaction between the compounds of formulae (VI) and (VII) is triethylamine.

13. Synthesis process according to any one of claims 1 to 12, **characterised in that** the amount of acid used in the reaction for cyclisation of the compound of formula (V) is from 5 to 15 moles per mole of compound of formula (V).

14. Synthesis process according to any one of claims 1 to 13, **characterised in that** the temperature of the reaction for cyclisation of the compound of formula (V) in an acid medium is from 0 to 40°C.

15. Synthesis process according to any one of claims 1 to 14, **characterised in that** the acid used for cyclisation of the compound of formula (V) is concentrated sulphuric acid.

16. Synthesis process according to claim 15, **characterised in that** the amount of concentrated sulphuric acid used in the reaction for cyclisation of the compound of formula (V) is from 1.5 to 3 millilitres per gram of (3,4-dimethoxyphenyl)acetic acid of formula (IV).

17. Process for the synthesis of ivabradine and pharmaceutically acceptable salts thereof, wherein the compound of formula (IV) is converted into the compound of formula (I) in accordance with the process of claim 1 and then the compound of formula (I) is converted into ivabradine.
